(19) **Europäisches Patentamt**
**European Patent Office**
**Office européen des brevets**

(11) **EP 1 517 709 B1**

(12) **EUROPEAN PATENT SPECIFICATION**

(45) Date of publication and mention
of the grant of the patent:
**11.06.2008 Bulletin 2008/24**

(51) Int Cl.:
***A61K 47/00*** (2006.01)

(21) Application number: **03730454.0**

(86) International application number:
**PCT/IL2003/000513**

(22) Date of filing: **16.06.2003**

(87) International publication number:
**WO 2004/000358 (31.12.2003 Gazette 2004/01)**

(54) **TRANSDERMAL DRUG DELIVERY SYSTEM**

TRANSDERMALES ARZNEIMITTELVERABREICHUNGSSYSTEM

SYSTEME D'ADMINISTRATION TRANSDERMIQUE DE MEDICAMENTS

(84) Designated Contracting States:
**CH DE FR GB LI NL**

(30) Priority: **20.06.2002 IL 15033402**

(43) Date of publication of application:
**30.03.2005 Bulletin 2005/13**

(73) Proprietor: **Sintov, Amnon**
**84965 Omer (IL)**

(72) Inventors:
• **SINTOV, Amnon**
**Omer 84965 (IL)**
• **GORODISCHER, Raphael**
**84965 Omer (IL)**

(74) Representative: **Schrell, Andreas**
**Gleiss Grosse Schrell & Partner**
**Patentanwälte Rechtsanwälte**
**Leitzstrasse 45**
**70469 Stuttgart (DE)**

(56) References cited:
**WO-A-01/70242          WO-A-02/09763**
**WO-A-94/07468          GB-A- 2 087 721**
**US-A- 5 314 915          US-B1- 6 206 920**

• **MOLINA I ET AL: "Protein release from physically crosslinked hydrogels of the PLA/PEO/PLA triblock copolymer-type" BIOMATERIALS, ELSEVIER SCIENCE PUBLISHERS BV., BARKING, GB, vol. 22, no. 4, 15 February 2001 (2001-02-15), pages 363-369, XP004226367 ISSN: 0142-9612**

Remarks:
The file contains technical information submitted after the application was filed and not included in this specification

**Description**

**Background of the Invention**

The present invention relates to a transdermal delivery system for local anesthetic, immunosuppresive and neurologically effective drugs, as well as for polypeptides and protein-based drugs. More particularly, the present invention relates to a transdermal delivery system for such drugs as granisetron, lidocaine, and cyclosporine in a transdermal drug delivery system (TDDS).

The present invention is a modification of the invention described and claimed in WO 02/09763.

In WO 02/09763 there is described and claimed a transdermal delivery system for analgesic, anti-pyretic and anti-inflammatory drugs comprising an analgesic, anti-pyretic or anti-inflammatory drug in combination with water-miscible tetraglycol and water for dissolving said drug in hydrogel form.

As stated, WO 02/09763 teaches a transdermal delivery system especially useful for non-steroidal-anti-inflammatory drugs.

It has now been found that not only non-steroidal-anti-inflammatory drugs can be effectively transported across the skin by the drug delivery system described in said Specification, but that many other types of active molecules may also be delivered transdermally utilizing a combination of water-miscible tetraglycol and water for dissolving such drugs in hydrogel form and this especially when said transdermal delivery system is in the form of a microemulsion. Thus it has now been discovered that by mixing a drug model and tetraglycol in a microemulsion, in a plain solution as well as in patches or hydrogels containing an ionized polymer, the obtained drug delivery system resulted in an enhanced percutaneous permeation thus increased the drug's potential of curing, healing or improving its therapeutic effect.

Thus according to the present invention there is now provided a transdermal delivery system for local anesthetic, immunosuppresive and neurologically effective drugs, as well as for polypeptides and protein-based drugs comprising a local anesthetic, immunosuppresive or neurologically effective drug, as well as a polypeptide or protein-based drug in combination with water-miscible tetraglycol and water for dissolving said drug in hydrogel form.

In preferred embodiments of the present invention said drug is selected from the group consisting of granisetron, lidocaïne, and cyclosporine.

The novel transdermal drug delivery system (TDDS) is preferably applied using a unilayer polymeric patch with adhesive margins, providing an effective and convenient mode of drug delivery.

In principle, transdermal administration of medications has several advantages: elimination of variations in plasma concentration after gastrointestinal absorption, elimination of hepatic first pass metabolism, and avoidance of gastrointestinal intolerance. The dermal administration of drugs may produce less gastrointestinal. (GI) adverse reactions as compared with the oral route, as it is assumed that some of the GI adverse effects are due to the local action of the drug (e.g., in stomach). The transdermal route of administration may be of particular significance in infants and in children because of their greater surface area to weight ratio. The epidermis of the full term neonate (but not that of the premature infant) is well developed and similar to that of an older child or adult. But the thinner skin with relatively rich blood supply of the infant and child may affect the pharmacokinetics of drugs administered by transdermal delivery systems, this has obvious therapeutic advantages, but it may have also toxic significance (the majority of cases of percutaneous drug toxicity have occurred in infants: aniline dye, hexachlorophene, iodine, and alcohol poisoning). Transdermal drug absorption can significantly alter drug kinetics depending on a number of factors, such as site of application, thickness and integrity of the stratum corneum epidermis, size of the molecule, permeability of the membrane of the transdermal drug delivery system, state of skin hydration, pH of the drug, drug metabolism by skin flora, lipid solubility, depot of drug in skin, alterations of blood flow in the skin by additives and body temperature.

While topical drug delivery systems have been used for centuries for the treatment of local skin disorders, the use of the skin as a route for systemic drug delivery is of relatively recent origin. Transdermal administration of drugs has been established in adults in relation to nitroglycerine, estrogens, scopolamine, and fentanyl. Although lacking adequate pediatric studies, scopolamine and fentanyl are often used in pediatric patients by this route. There are data on the pharmacokinetics and clinical use of transdermal administration of theophylline in human neonates: after a shingle application to the skin an hydrogel disc system resulted in therapeutic concentrations of theophylline for up to 3 days in neonates with apnea.

Many drugs are practically insoluble in water or slightly soluble even in their ionized form. Therefore, dissolution of active agents in topical and transdermal preparations usually requires incorporation of an alcohol. However, the use of solvents like alcohols in topical preparations may lead to precipitation of the drug on the skin upon evaporation of the solvent once spread over the skin area. In situations in which the application area is occluded, such as in transdermal patches, alcohol presence may cause a skin irritation and inflammatory conditions.

The present invention obviates this problem since it enables the preparation of transdermal delivery systems for drugs usually requiring alcohol for the dissolution thereof, utilizing water-miscible tetraglycol instead of the standard alcohols used heretofor.

Thus, the present invention also provides a transdermal delivery system for an alcohol-miscible drug comprising an alcohol-miscible local anesthetic, immunosuppresive or neurologically effective drug, as well as a polypeptide or protein-based drug in combination with water-miscible tetraglycol and water for dissolving said drug in hydrogel form wherein said transdermal delivery system is in the form of a microemulsion.

Another aspect of the present invention comprises the method of preparing transdermal drug delivery systems. This method includes preparation of 'easy-to-make' patches containing the drug, tetraglycol and other ingredients, which adhere spontaneously to the skin surface. The manufacturing method of transdermal patches of the present invention is unique by the virtue of the guar-based polymer to solidify the drug-containing liquid within few minutes to a patch at any desired size, shape and thickness. The manufacturing of the composition can be designed in such a way that once the polymer is dispersed in a liquid mixture containing the drug in a tetraglycol-containing microemulsion, the mixture is molded to a patch - a process that takes few minutes at the best case to several hours at the worst. The obtained patch is self-adhesive to the skin surface, requiring only a covering sheet with adhesive margins to occlude the system from any kind of evaporation or contamination during treatment.

## Detailed Description of the Invention

It should be defined, that by the term "comprising" as used in the present invention is meant that various other inactive ingredients, compatible drugs and medicaments can be employed in the compositions as long as the critical tetraglycol.or ionized polymers are present in the compositions and are used in the manner disclosed.

All percentages herein are by weight unless otherwise specified.

As stated hereinbefore, the transdermal delivery systems of the present invention comprise an effective amount of:

(a) a local anesthetic, immunosuppresive or neurologically effective drug, as well as a polypeptide or protein-based drug, or combinations of such drugs; and

(b) the water-miscible tetraglycol (TG), which can be mixed with any portion of water.

In addition, the transdermal systems of the present invention preferably include further components as follows:

(c) in the case where the composition according to the invention is a gel, soft or hard patch, stabilizers or shape-forming agents are selected from the group consisting of ionized polymers such as cationized guar gum, cellulose derivatives, acrylic polymers, polysaccharides, lipids, proteins, and polyhydroxy compounds. The average molecular weight of these polymers can vary from 5,000 to 500,000 daltons. The preferred polymer for the transdermal patch of the present invention is hydroxypropyl guar hydroxypropyltrimonium chloride (guar-based polymer, GP);

(d) in case that the composition is an emulsion, the oil phase comprises at least one ester selected from the group consisting of monoglycerides, diglycerides and triglycerides of monocarboxylic acids selected from the group consisting of saturated monocarboxylic acids and monocarboxylic acids containing ethylenic unsaturation. The emulsion is prepared by using pharmaceutically acceptable emulsifiers containing at least one esterified carboxylic group in its structure.

Further preferred components include:

(e) poly- or oligo- hyroxy compounds or their derivatives as co-solvents. These compounds can be selected from the group of polyalkylene glycols, poloxamers, and di- or tri- ethylene glycol ethyl ethers;

(f) skin penetration enhancers selected from nonionic surfactants consisting of sorbitan sesquioleate, cetostearyl alcohol, polysorbate 60, sorbitan monostearate, sorbitan monooleate, and a preferred combination of polyoxyethylene 23 lauryl alcohol and glyceryl mono/di-oleate;

(g) safe and effective preservatives such as parabens, benzyl alcohol and benzoic acid pH adjusting agents such as triethanolamine, citric and lactic acid may also be included in the composition.

Preferred compositions according to the present invention are pharmaceutically accepted and easy-to-apply skin-adhesive systems containing NSAIDs as active ingredients. More particularly, these systems composed of tetraglycol (glycofurol, tetrahydrofurfuryl alcohol polyethyleneglycol ether) (TG) and an ionized polymer such as hydroxypropyl guar hydroxypropyltrimonium chloride (guar-based polymer, GP), which assist in dissolving or solubilizing the active materials in a hydrogel form, and facilitate their penetration through the lipophilic strata of the skin.

While the invention will now be described in connection with certain preferred embodiments in the following examples and with reference to the accompanying figures so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

In the drawings:

Fig. 1 is a graphical representation of the percutaneous penetration of granisetron.

Fig. 2, 3, 4a, 4b and 5 are graphical representations of the percutaneous penetration of lidocaine.

Fig. 6a and 6b are graphical representations of the concentrations of cyclosporine found in the epidermal (A) and dermal (B) layers and

Fig. 7 is a graphical representation showing the partitioning of cyclosporine between the dermis and epidermis.

**Example I - Granisetron**

This example is a study performed *in vitro* using porcine ear skin as described below:

*Diffusion cells:* The permeability of granisetron through porcine skin was measured *in-vitro* with a Franz diffusion cell system. The diffusion area was 1.767 $cm^2$ (15 mm diameter orifice), and the receptor compartment volumes varied between 11 to 12 ml. The solutions on the receiver side were stirred by externally driven, teflon-coated magnetic bars.

*Skin preparation:* Full-thickness porcine skin was excised from fresh ears of slaughtered white pigs (breeding of Landres and Large White, locally grown in Kibbutz Lahav, Israel). Skin sections (about 2X2 cm) were cut and subcutaneous fat was removed from the skin sections with a scalpel. Transepidermal water loss measurements (TEWL, Dermalab® Cortex Technology, Hadsund, Denmark) was performed and only those pieces that the TEWL levels were within specification (<10 $g/m^2h$) were mounted in the diffusion cells.

*Permeation study:* 200 mg specimens of three granisetron preparations [5% aqueous solution, 5% in tetraglycol-containing microemulsion, and 5% granisetron in a same microemulsion gelled with ionized polymer - see Table 1 below] were applied on the skin. The receiver solutions (PBS, pH 7.4) were sampled at predetermined time intervals, and the cells were replenished to their marked volumes with fresh buffer solution. Samples were kept at -20oC until analyzed by HPLC.

*Calculations:* As a result of a large volume sampling from the receiver solution and replacing with equal volumes, the solution is constantly diluted. Taking this into account, cumulative drug permeation ($Q_t$) is calculated from the following equation:

$$Q_t = V_r C_t + \sum_{i=0}^{t-1} V_s C_i$$

where $C_t$ is the drug concentration of the receiver solution at each sampling time, $C_i$ is the drug concentration of the *i*-th sample, and $V_r$ and $V_s$ are volumes of the receiver solution and the sample, respectively. Data were expressed as the cumulative granisetron permeation per unit of skin surface area, $Q_t/S$ (S=1.767$cm^2$).

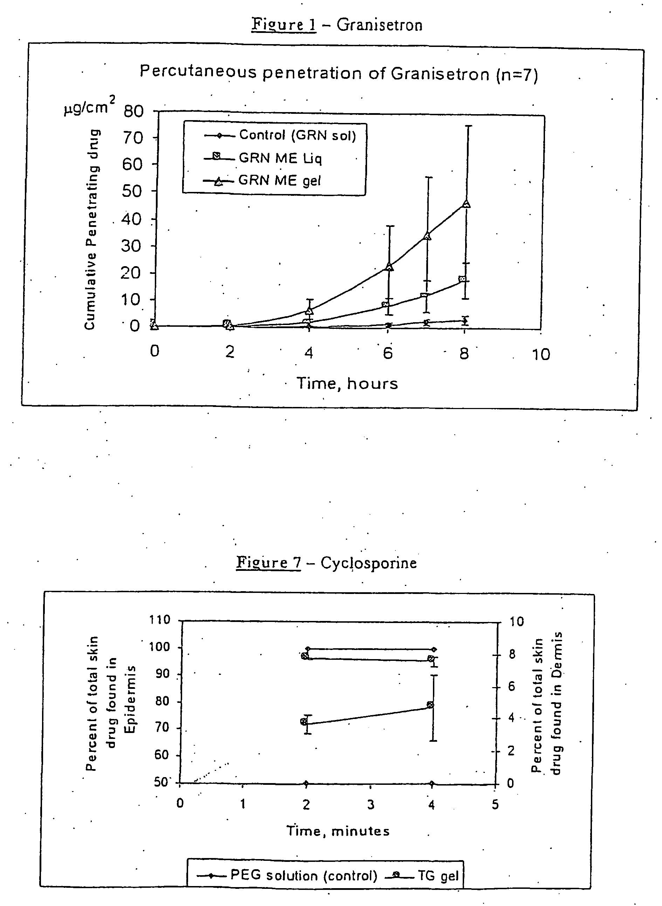

*Results:* Figure 1 shows the differences found between the 3 formulations tested. The percutaneous penetration of granisetron increased significantly when a tetraglycol-containing microemulsion was applied on the skin (3.84 $\mu g/cm^2/hr$ vs. 0.60 $\mu g/cm^2/hr$), however, it increased even more (up to 10.06 $\mu g/cm^2/hr$) when the guar-based ionized polymer was incorporated.

T**able 1** - Granisetron

| | Formulations | | |
|---|---|---|---|
| Ingredients | 5% GRN in microemulsion gel | 5% GRN in microemulsion liquid | 5% GRN in an aqueous solution |
| | | | |
| Granisetron | 0.125 g | 0.125 g | 0.05 g |
| Distilled water | 0.5 ml | 0.5 ml | 0.95 ml |

(continued)

| | Formulations | | |
|---|---|---|---|
| Ingredients | 5% GRN in microemulsion gel | 5% GRN in microemulsion liquid | 5% GRN in an aqueous solution |
| Tetraglycol (TG) | 0.375 g | 0.875 g | ----- |
| Isopropyl palmitate | 0.25 g | 0.25 g | ----- |
| Arlacel 186 | 0.25 g | 0.25 g | ----- |
| Chremophor RH40 | 0.50 g | 0.50 g | ----- |
| Jaguar C-162 | 0.50 g | ----- | ----- |

• GRN = granisetron
• Chremophor RH40 = polyoxyl 40 hydrogenated castor oil USP24/NF19 (PEG-40 hydrogenated castor oil) [Manufacturer: BASF, Germany].
• Arlacel 186 = glyceryl oleate [Manufacturer: Uniqema, UK].
• Jaguar C-162 = guar-based polymer (Guar gum, 2-hydroxypropyl 2-hydroxy-3-(trimethylammonio)propyl ether chloride; CTFA/INCI name: Hydroxypropyl guar hydroxypropyltrimonium chloride) [Manufacturer: Rhone-Poulenc, France].

**Example II - Lidocaine**

This example includes experiments that were performed *in vitro* using excised rat skin and *in vivo* in rats as described below:

*In vitro:* The diffusion testing, skin preparation, permeation and calculations were performed exactly as described for granisetron, except that the skin was taken from the abdominal side of Sprague-Dawley rats. The rats (around 500 g) were killed by aspiration of ethyl ether vapors and the abdominal hair was then trimmed off with a hair clipper. Sections of full-thickness abdominal skin were excised from the fresh carcasses of the animals. Subcutaneous fat was removed with a scalpel, and the skin sections were mounted in the diffusion cells. The skin was placed with the stratum corneum facing up on the receiver chambers, and then the donor chambers were clamped in place. The excess skin was trimmed off and the receiver chamber, defined as the side facing the dermis, was filled with phosphate buffer (4mM, pH=7.4).

*In vivo*: The *in-vivo* skin penetration was evaluated in anesthetized rats. Anesthetized (15 mg/kg pentobarbital sodium *i.p.*) rats (Sprague-Dawley, 250-300g) were placed on their back, the abdominal hair was trimmed off, and the skin was washed gently with distilled water. Drug-containing preparations were applied on a restricted skin surface (4.9 $cm^2$).. Blood samples were taken from the tail vein into heparanized tubes at t=0, 1, 2, 3, 4, 5, and 6 hours from the time of drug application. In a separated study 18 anesthetized rats were applied with (a) 2.5% lidocaine base in tetraglycol-containing microemulsion gelled with guar-based ionized polymer [n=9], and (b) a commercial product known as EMLA 5% cream (Astra) containing 2.5% lidocaine (as base) and 2.5% prilocaine [n=9]. After 15, 30 and 60 minutes of *in vivo* study (3 animals were designated for each time and for each group), the products were removed from the animals and they were killed by aspiration of ethyl ether. The drug-exposed skin areas were swabbed 3-4 times with 3 layers of gauze pads, washed for 30 seconds with running water, wiped carefully and harvested from the animals. The excised skin sections were rinsed with phosphate buffer (pH=7.4), and heat-separated (60°C, 60 seconds) to its epidermal and dermal layers. Each layer was cut to small pieces and inserted in 2-ml vials. The tissue in each vial was extracted by ethyl alcohol. Each extraction was performed by incubation in a 40°C shaking water bath (150 rpm) for 1 hour. The extracts were injected into the HPLC system.

*Formulations tested in-vitro and in-vivo* (see Table 2):

(a) 2.5% lidocaine (as base) in tetraglycol-containing microemulsion liquids
(b) 2.5% lidocaine (as base) in tetraglycol-containing microemulsion gels prepared with guar-based ionized polymer.
(c) Commercial product as reference: EMLA 5% cream (Astra) containing 2.5% lidocaine (as base) and 2.5% prilocaine.

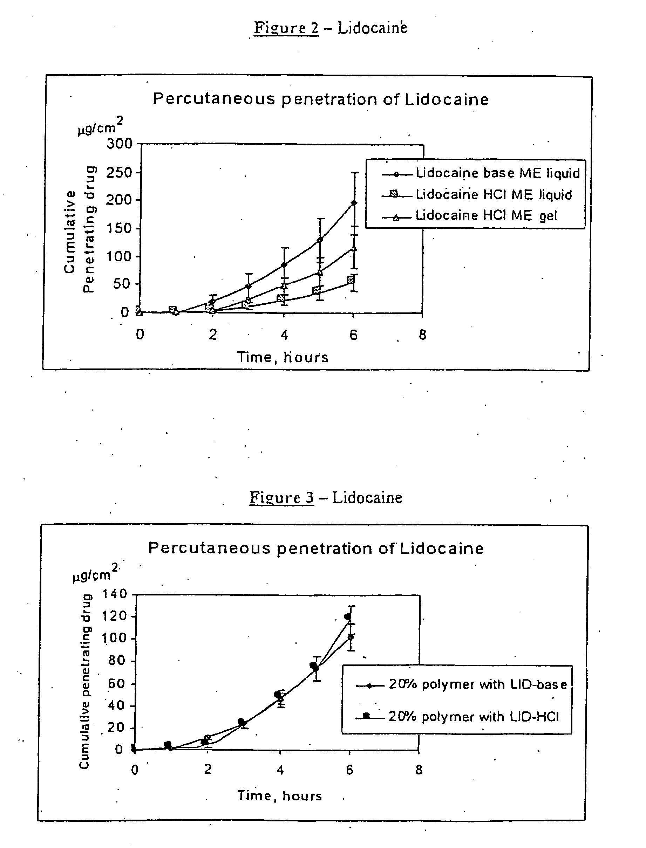

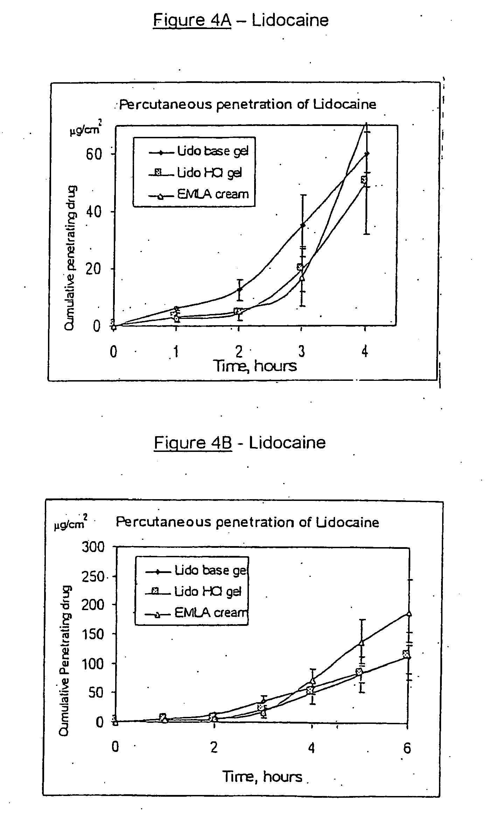

*Results*: Figure 2 presents the profiles of percutaneous penetration kinetics of lidocaine hydrochloride delivered from tetraglycol-containing microemulsions. It can be seen that by combining the ionized polymer, the penetration through the skin was accelerated significantly. This increase in the drug penetration may be explained by neutralization of lidocaine HCl to lidocaine base by the polymer, thus raising drug lipophilicity. The relatively higher penetration of lidocaine base from a microemulsion liquid, which is also demonstrated in the Figure, supports this hypothesis. Nevertheless, as observed in Figures 3 and 4, there has been found some difference between the penetrations of hydrochloride and the base molecules in gels. A higher penetration of the base was repeatedly noted only during the first hours of gel application, implying that the base is superior over the hydrochloride in advancing the onset time of the local anesthetic effect. It has also been found (Figure 4) that more lidocaine base penetrated from the gel than from the patented commercial EMLA cream (also containing lidocaine basse).

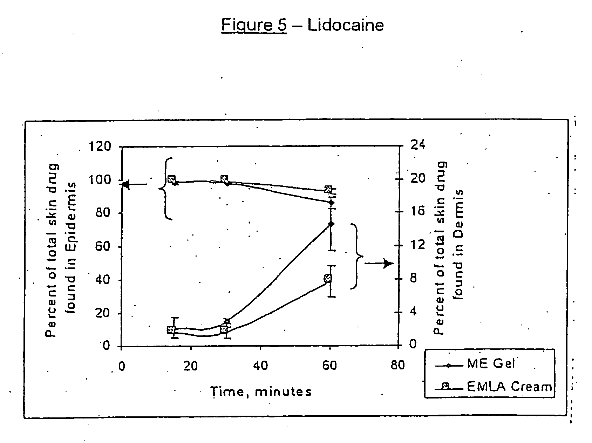

While the *in vitro* penetration showed quantitative amounts of penetrating drug, the *in vivo* study failed to show systemic plasma levels that were above the limit of HPLC assay quantitation. To extrapolate the penetration rates of drug quantities across the skin (fuxes), it was necessary, therefore, to monitor the drug in the skin layers. Figure 5 presents the partitioning of lidocaine between the dermis and the epidermis after an *in vivo* study. The figure clearly shows that within an hour, a relatively higher portion of the cutaneous drug accumulated in the dermis of skin treated with the tetraglycol gel. In contrast, EMLA cream delivered more drug to the epidermis with a lower drug partitioning in the dermis, indicating a slower penetration of lidocaine across the full-thickness skin that results in lower drug levels in the subcutis and the surrounding tissues.

**Table 2 - Lidocaine**

|  | Formulations | | |
|---|---|---|---|
| Ingredients | 2.5% LID-base in microemulsion gel | 2.5% LID-HCl in microemulsion gel | 2.5% lidocaine (HCl or base) in microemulsion liquid |
|  |  |  |  |
| Lidocaine | 0.125 g as base | 0.125 g as base | 0125 g as base |
| Distilled water | 1 ml | 1 ml | 1 ml |
| Tetraglycol (TG) | 0.875 g | 0.875 g | 1.875 g |
| Isopropyl palmitate | 0.5 g | 0.5 g | 0.5 g |
| Arlacel 186 | 0.5 g | 0.5 g | 0.5 g |
| Chremophor RH40 | 1.0 g | 1.0 g | 1.0 g |
| Jaguar C-162 | 1.0 g | 1.0 g | --------- |

• LID = Lidocaine
• Chremophor RH40 = polyoxyl 40 hydrogenated castor oil USP24/NF19 (PEG-40 hydrogenated castor oil) [Manufacturer: BASF, Germany].
• Arlacel 186 = glyceryl oleate [Manufacturer: Uniqema, UK].
• Jaguar C-162 = guar-based polymer (Guar gum, 2-hydroxypropyl 2-hydroxy-3-(trimethylammonio)propyl ether chloride; CTFA/INCI name: Hydroxypropyl guar hydroxypropyltrimonium chloride) [Manufacturer: Rhone-Poulenc, France].

### Example III - Cyclosporine

This example includes experiments that were performed *in vivo* in rats as described below:

The *in-vivo* skin penetration was evaluated in anesthetized rats. Anesthetized (15 mg/kg pentobarbital sodium *i.p.*) rats (Sprague-Dawley, 250-300g) were placed on their back, the abdominal hair was trimmed off, and the skin was washed gently with distilled water. Drug-containing preparations were applied on a restricted skin surface (4.9 cm$^2$). After 2 and 4 hours of *in vivo* study (3 animals were designated for each time and for each group), the products were removed from the animals and they were killed by aspiration of ethyl ether. The drug-exposed skin areas were swabbed 3-4 times with 3 layers of gauze pads, washed for 30 seconds with running water, wiped carefully and harvested from the animals. The excised skin sections were rinsed with phosphate buffer (pH=7.4), and heat-separated (60°C, 60 seconds) to its epidermal and dermal layers. Each layer was cut to small pieces and inserted in 2-ml vials. The tissue in each vial was extracted by ethyl alcohol. Each extraction was performed by incubation in a 40°C shaking water bath (150 rpm) for 1 hour. The extracts were injected into the HPLC system.

*Test formulations* (see Table 3):

(a) 0.5% cyclosporine in propylene glycol/polyethylene glycol (PEG) 400
(b) 0.5% cyclosporine in tetraglycol-containing gel prepared with guar-based ionized polymer.

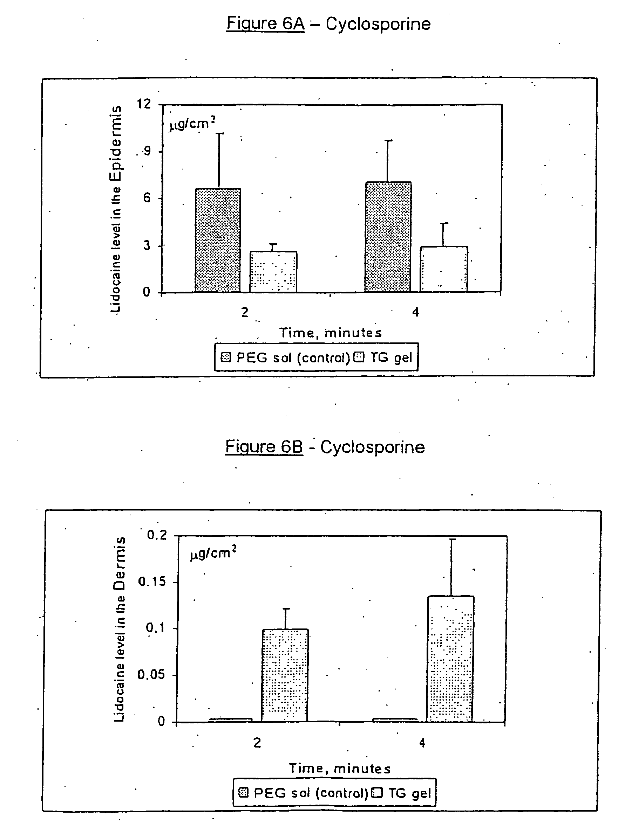

*Results*: Figure 6 presents the concentrations of cyclosporine found in the epidermal (A) and dermal (B) layers. After application of 0.5% drug solution containing propylene glucol as a commonly used enhancer, it was obvious that cyclosporine massively accumulated in the epidermis while no drug was detected in the dermis. In contrast, a gel containing 0.5% cyclosporine in tetraglycol delivered the drug into the dermis at levels of upto 100 ng/cm$^2$ skin surface area after 4 hours. No difference was noted between gels containing 20% and 10% ionized polymer (Table 3). Figure 7 illustrates the results differently by showing the partitioning of cyclosporine between the dermis and the epidermis. The figure clearly shows that already after 2 hours, a relatively high portion (about 4%) of the cutaneous drug accumulated in the dermis of skin treated with the tetraglycol-ionized polymer. The portion of drug entered into the dermis was increasingly growing during the next 2 hours. No drug fraction was found in the dermis after a topical solution of PEG-propylene glycol was used.

**Table 3 - Cyclosporine**

| | Formulations | | |
|---|---|---|---|
| Ingredients | TG gel 20% polymer | TG gel 10% polymer | PEG solution |
| | | | |
| Cyclosporine | 50 mg | 50 mg | 50 mg |
| Propylene glycol | -------- | -------- | 1.0 g |
| PEG 400 | -------- | -------- | 8.95 g |
| Distilled water | 3 ml | 3 ml | -------- |
| Tetraglycol (TG) | 4.95 g | 5.95 g | -------- |
| Jaguar C-162 | 2.0 g | 1.0 g | -------- |
| • PEG 400 = polyethylene glycol 400 (or polyoxyethylene glycol 400, Macrogol 400) <br> • Jaguar C-162 = guar-based polymer (Guar gum, 2-hydroxypropyl 2-hydroxy-3-(trimethylammonio)propyl ether chloride; CTFA/INCI name: Hydroxypropyl guar hydroxypropyltrimonium chloride) [Manufacturer: Rhone-Poulenc, France]. | | | |

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

**Claims**

1.  A transdermal delivery system for local anesthetic, immunosuppressive and neurologically effective drugs, as well as for polypeptides and protein-based drugs, comprising a local anesthetic, immunosuppressive or neurologically effective drug, as well as a polypeptide or protein-based drug in combination with water-miscible tetraglycol and water for dissolving said drug in hydrogel form, wherein said transdermal delivery system is in the form of a microemulsion.

2.  A transdermal delivery system according to claim 1, further comprising an ionized polymer.

3.  A transdermal delivery system according to claim 2, wherein said ionized polymer is selected from the group consisting of cationized guar gum, cellulose derivatives, acrylic polymers, polysaccharides, lipids, proteins and polyhydroxy compounds.

4. A transdermal delivery system according to claim 2, wherein said ionized polymer is a guar-based polymer, which serves as a gelling agent for said composition.

5. A transdermal delivery system according to claim 3, wherein said guar-based polymer is hydroxypropyl guar hydroxypropyltrimonium chloride.

6. A transdermal delivery system according to claim 1, wherein said drug is selected from the group consisting of granisetron, lidocaine, and cyclosporine.

7. A transdermal delivery system according to claim 1, wherein said transdermal delivery system is in the form of a hydrogel patch.

8. A transdermal delivery system according to claim 1, further comprising a skin penetration enhancer.

9. A transdermal delivery system according to claim 8, wherein said skin penetration enhancer is a non-ionic surfactant.

10. A transdermal delivery system according to claim 9, wherein said non-ionic surfactant is selected from the group consisting of sorbitan sesquioleate, cetostearyl alcohol, polysorbate 60, sorbitan monostearate, sorbitan monooleate, polyoxyethylene 23 lauryl alcohol, glyceryl mono/di-oleate and mixtures thereof.

**Patentansprüche**

1. Transdermales Verabreichungssystem für lokalanästhetische, immunosuppressive und neurologisch wirksame Arzneimittel, sowie für auf Polypeptiden und auf Proteinen basierende Arzneimittel, umfassend ein lokalanästhetisches, immunosuppressives oder neurologisch wirksames Arzneimittel, sowie ein polypeptid- oder proteinbasiertes Arzneimittel in Kombination mit wassermischbarem Tetraglycol und Wasser zum Auflösen des Arzneimittels in Hydrogelform, wobei das transdermale Verabreichungssystem in Form einer Mikroemulsion vorliegt.

2. Transdermales Verabreichungssystem nach Anspruch 1, des Weiteren umfassend ein ionisiertes Polymer.

3. Transdermales Verabreichungssystem nach Anspruch 2, wobei das ionisierte Polymer ausgewählt ist aus der Gruppe bestehend aus kationisiertem Guargummi, Cellulosederivaten, Acrylpolymeren, Polysacchariden, Lipiden, Proteinen und Polyhydroxy-Verbindungen.

4. Transdermales Verabreichungssystem nach Anspruch 2, wobei das ionisierte Polymer ein auf Guar basierendes Polymer ist, das als ein Geliermittel für die Zusammensetzung dient.

5. Transdermales Verabreichungssystem nach Anspruch 3, wobei das auf Guar basierende Polymer Hydroxypropyl-Guar-Hydroxypropyltrimoniumchlorid ist.

6. Transdermales Verabreichungssystem nach Anspruch 1, wobei das Arzneimittel ausgewählt ist aus der Gruppe bestehend aus Granisetron, Lidocain und Cyclosporin.

7. Transdermales Verabreichungssystem nach Anspruch 1, wobei das transdermale Verabreichungssystem in Form eines Hydrogel-Pflasters vorliegt.

8. Transdermales Verabreichungssystem nach Anspruch 1, des Weiteren umfassend ein die Hautdurchdringung förderndes Mittel.

9. Transdermales Verabreichungssystem nach Anspruch 8, wobei das die Hautdurchdringung fördernde Mittel ein nichtionischer oberflächenaktiver Stoff ist.

10. Transdermales Verabreichungssystem nach Anspruch 9, wobei der nichtionische oberflächenaktive Stoff ausgewählt ist aus der Gruppe bestehend aus Sorbitansesquioleat, Cetostearylalkohol, Polysorbat 60, Sorbitanmonostearat, Sorbitanmonooleat, Polyoxyethylen 23-Laurylalkohol, Glycerylmono-/dioleat und Gemischen davon.

**Revendications**

1. Système d'administration transdermique pour médicaments anesthésiques locaux, immunosuppressifs et efficaces sur le plan neurologique, ainsi que pour médicaments à base de polypeptides et de protéines, comprenant un médicament anesthésique local, immunosuppressif ou efficace sur le plan neurologique, ainsi qu'un médicament à base de polypeptides ou de protéines en combinaison avec du tétraglycol miscible dans l'eau et de l'eau pour dissoudre ledit médicament sous forme d'hydrogel, ledit système d'administration transdermique étant sous la forme d'une micro-émulsion.

2. Système d'administration transdermique selon la revendication 1, comprenant en outre un polymère ionisé.

3. Système d'administration transdermique selon la revendication 2, dans lequel ledit polymère ionisé est choisi dans le groupe constitué de gomme de guar rendue cationique, de dérivés de cellulose, de polymères acryliques, de polysaccharides, de lipides, de protéines et de composés polyhydroxy.

4. Système d'administration transdermique selon la revendication 2, dans lequel ledit polymère ionisé est un polymère à base de guar, qui sert d'agent gélifiant pour ladite composition.

5. Système d'administration transdermique selon la revendication 3, dans lequel ledit polymère à base de guar est du chlorure d'hydroxypropyltrimonium d'hydroxypropyl-guar.

6. Système d'administration transdermique selon la revendication 1, dans lequel ledit médicament est choisi dans le groupe constitué du granisétron, de la lidocaïne et de la cyclosporine.

7. Système d'administration transdermique selon la revendication 1, dans lequel système d'administration transdermique est sous la forme d'un patch d'hydrogel.

8. Système d'administration transdermique selon la revendication 1, comprenant en outre un agent favorisant la pénétration de la peau.

9. Système d'administration transdermique selon la revendication 8, dans lequel ledit agent favorisant la pénétration de la peau est un agent tensio-actif non ionique.

10. Système d'administration transdermique selon la revendication 9, dans lequel ledit agent tensio-actif non ionique est choisi dans le groupe constitué du sesquioléate de sorbitane, de l'alcool cétostéarylique, du polysorbate 60, du monostéarate de sorbitane, du monooléate de sorbitane, de l'alcool polyoxyéthylène 23 laurique, du mono/di-oléate de glycéryle et de mélanges de ceux-ci.

Figure 1 – Granisetron

Percutaneous penetration of Granisetron (n=7)

Figure 7 – Cyclosporine

Figure 2 – Lidocaine

Percutaneous penetration of Lidocaine

Legend:
—◇— Lidocaine base ME liquid
—▨— Lidocaine HCl ME liquid
—△— Lidocaine HCl ME gel

Figure 3 – Lidocaine

Percutaneous penetration of Lidocaine

Legend:
—◆— 20% polymer with LID-base
—■— 20% polymer with LID-HCl

## Figure 4A – Lidocaine

Percutaneous penetration of Lidocaine

## Figure 4B - Lidocaine

Percutaneous penetration of Lidocaine

## Figure 5 – Lidocaine

## Figure 6A – Cyclosporine

## Figure 6B - Cyclosporine

**EP 1 517 709 B1**

### REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0209763 A